# EUROPEAN PATENT APPLICATION

(11) **EP 2 001 200 A2**
(43) Date of publication of application: **10.12.2008**
(21) Application number: 08251925.7
(22) Date of filing: 03.06.2008
(51) Int. Cl.: H04L 29/06

(54) **Controlling vehicular electronics devices using physiological signals**

(30) Priority: 04.06.2007 KR 20070054497
(71) Applicant: Lee, Min Hwa, Gangnam-gu Seoul 135-787 (KR)
(72) Inventor: Lee, Min Hwa, Gangnam-gu Seoul 135-787 (KR)
(74) Representative: Harris, Ian Richard

(57) **Abstract**

A vehicle network system including at least one sensor (132-138), a network server (120), and a at least one vehicle network device (240-252) is provided. The at least one sensor (132-138) is arranged to generate physiological signals of a user, which are received and processed by the network server (120) is configured to receive and process to generate status data indicating the physical condition of the user. The vehicle network device is configured to control the one or more vehicular electronics devices based on the status data from the network server.

## Description

### TECHNICAL FIELD

The present disclosure relates generally to electronic devices, and more particularly to controlling vehicular electronics devices based on physiological signals.

### BACKGROUND

Motor vehicles typically include a variety of electronic devices for convenience and safety. For example, some cars and other vehicles now provide automatic climate systems to maintain a set temperature for the driver and passengers. Automatic lighting systems also provide a convenient way to illuminate the interior of the vehicle and control external lights. Convenience features such as seat adjustments, steering wheel adjustments, automatic mirrors, etc., provide additional convenience for drivers and passengers. Other features such as intelligent airbags are able to discriminate children from adults to prevent deployment for further safety.

In addition to these convenience features, vehicle manufacturers are providing advanced electronics for added convenience. For example, automobiles are often sold with advanced entertainment and communications systems, such as CD players, DVD players, navigation systems, mobile phone connections, etc. Each of these features, however, typically requires a separate control mechanism for user input or control. That is, these features generally require users to manipulate input mechanisms such as dials, keys, buttons, keypads, etc. for control. Such input mechanisms are often complex and may even lead to accidents by distracting drivers while driving.

### SUMMARY

The present disclosure provides a system and a method for controlling vehicular electronics devices based on physiological signals of one or more users. A plurality of sensors is provided to detect physiological signals from the users. A network server analyzes the detected physiological signals to generate status information indicating the health or a condition of the user. Based on the user's status information, control signals related to one or more vehicular electronics devices are generated to allow automatic control of these devices.

In accordance with one aspect of an embodiment of the present invention, a vehicle network system includes one or more sensors, a network server, and a vehicle network device. The one or more sensors are arranged to generate signals based on the physiological health of a user. The network server is configured to receive and process the physiological signals from the one or more sensors to generate status data indicating the physical condition of the user. Based on the status data from the network server, the vehicle network device is configured to control one or more vehicular electronics devices.

In accordance with another aspect of an embodiment of the present invention, a vehicle network system for controlling vehicular electronics devices includes a plurality of network servers and a vehicle network device. Each of the plurality of network servers is configured to receive and process physiological signals from one or more sensors to generate status data indicating the physical condition of a user. The vehicle network device is configured to control one or more vehicular electronics devices based on the status data from the plurality of network servers.

In accordance with another aspect of an embodiment of the present invention, a method for controlling vehicular electronics devices includes generating physiological signals of a user. The physiological signals are processed to generate status data indicating the health or a physical condition of the user. Based on this status data, control signals that are specific to one or more vehicular electronics devices are generated. The device specific control signals are provided to the one or more vehicular electronics devices to control the one or more vehicular electronics devices.

In accordance with still another aspect of an embodiment of the present invention, a method for controlling vehicular electronics devices includes receiving and processing, at least one terminal of a plurality of network terminals, physiological signals from one or more sensors to generate status data indicating the physical condition of a user. A vehicular network device is provided to control one or more vehicular electronics devices based on the status data of the user from the plurality of network servers.

### BRIEF DESCRIPTION OF THE DRAWINGS

The foregoing and other objects and features of the present disclosure will become more fully apparent from the following description and appended claims, taken in conjunction with the accompanying drawings. Understanding that these drawings depict only typical embodiments in accordance with the present disclosure and are, therefore, not to be considered limiting of its scope, the present disclosure will be described with additional specificity and detail through use of the accompanying drawings in which:

FIG. 1 shows a block diagram of a system connecting a vehicle area network (VAN) and a body area network (BAN) server to automatically control vehicular electronics devices in the vehicle area network (VAN) in accordance with one embodiment of the present disclosure.

FIG 2 shows a more detailed diagram of a system for controlling vehicular electronics devices in a vehicle in response to sensors in accordance with one embodiment of the present disclosure.

FIG 3 illustrates a block diagram in accordance with one embodiment, where the body area network (BAN) server is a mobile terminal

FIG 4 illustrates a method for controlling a plurality of vehicular electronics devices by using a mobile terminal in accordance with one embodiment of the present disclosure.

FIG 5 shows a flowchart of a method performed by a vehicle area network (VAN) to control vehicular electronics devices in accordance with one embodiment of the present disclosure.

### DETAILED DESCRIPTION

It will be readily understood that the components of the present disclosure, as generally described and illustrated in the Figures herein, could be arranged and designed in a wide variety of different configurations. Thus, the following more detailed description of the embodiments of apparatus and methods in accordance with the present disclosure, as represented in the Figures, is not intended to limit the scope of the present claims, but is merely representative of certain examples of presently contemplated embodiments in accordance with the present disclosure. The presently described embodiments will be best understood by reference to the drawings, wherein like parts are designated by like numerals throughout.

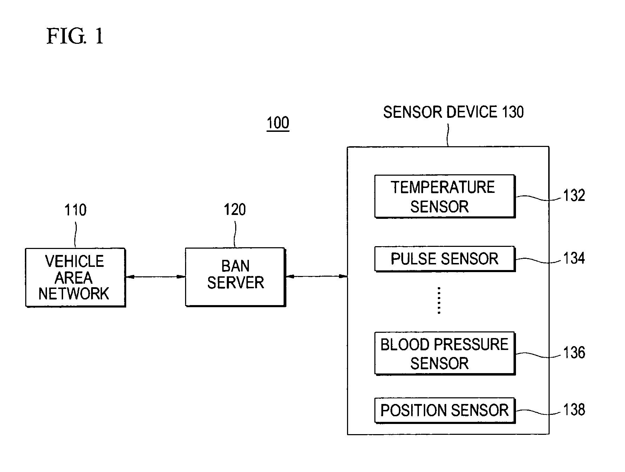

FIG 1 shows a block diagram of a system 100 connecting a vehicle area network (VAN) 110 and a body area network (BAN) server 120 (e.g., network server, mobile terminal, mobile phone, PDA or similar device.) to automatically control vehicular electronics devices based on physiological signals from a sensor device 130 in accordance with one embodiment. The sensor device 130 includes a plurality of sensors 132, 134, 136, and 138 that are configured to generate signals indicating a user's physiological state or condition. Other or alternate sensors for measuring other physiological states or conditions of a user may be included in the sensor device 130 as well. The BAN server 120 is coupled, via either a wired or wireless connection, to the sensor device 130 to receive physiological signals from it. Upon receiving physiological signals, the BAN server 120 processes the signals to generate status information (e.g., data) indicating the condition or state of the user. The VAN 110 is coupled to receive the status information signals from the BAN server 120, via a wired or wireless connection, to control various vehicular electronics devices and control units in the VAN 110. To identify the BAN server 120, the BAN server 120 is associated with a unique ID, which is provided to the VAN 110 along with the user's status information.

The sensor device 130 includes a plurality of sensors that detect various physiological parameters of an individual, such as heart rate, pulse rate, beat-to-beat heart variability, EKG or ECG, respiration rate, skin temperature, core body temperature, heat flow off the body, galvanic skin response, blood pressure, body fat, oxygen consumption, body position, pressure on muscles and bones, UV radiation exposure and absorption, etc. In the illustrated embodiment, the sensor device 130 includes sensors such as temperature sensor 132 for detecting the temperature of a user or vehicle interior, a pulse sensor 134 for detecting the pulse rate of the user, a blood pressure sensor 136 for detecting the blood pressure of the user, a position sensor 138 for detecting the position (e.g., vertical, horizontal, etc.) of various body parts of the user, etc. As set forth above, other or alternate sensors for measuring other physiological states or conditions may be included in the sensor device 130.

The sensors 132, 134, 136, and 138 may be worn, attached (e.g., as a patch), or located in proximity to a user to detect and transmit physiological signals to the BAN server 120. In selected embodiments, the physiological sensors 132, 134, 136, and 138 may generate signals that are interfaced to standard wired or wireless network platforms (not shown) that may provide various capabilities, including but not limited to computational, storage, and communication capabilities. To this end, the multiple physiological sensors 132, 134, 136 and 138 may be integrated with a sensor board capable of providing on-sensor processing capability, and communicates with a standard wireless network platform through, e.g., serial interfaces. In the case of a wireless BAN server 120, the sensor device 130 may be preferably equipped with a sensor board, including transmitters capable of transmitting detected signals to the BAN server 120 via the platform using a wireless interface protocol, e.g., ZigBee, Blue-tooth, RF communications, etc. In one embodiment, each of the sensors 132 to 138 has a sensor ID and transmits the sensor ID to the BAN server 120 with detected signals to allow the BAN server 120 to identify the source sensor and the received signals.

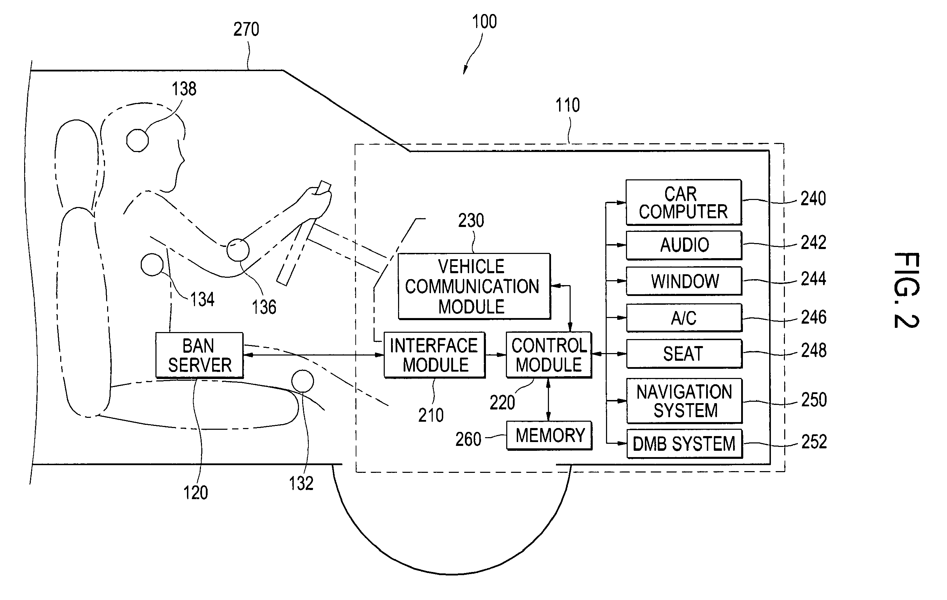

FIG 2 shows a more detailed diagram of the system 100 for controlling the VAN 110 in a vehicle 270 via the BAN server 120 in response to the sensors 132 to 138 in accordance with one embodiment. In this embodiment, the sensors 132 to 138 are placed on the driver's body to detect and generate signals corresponding to the driver's condition or state. As described above, the BAN server 120 receives physiological signals from the sensors 132, 134, 136, and 138 integrated with a sensor board with transmitting capability. Each of the sensors 132, 134, 136, and 138 may share standard wired or wireless network platforms that may act as a node to communicate with the BAN server 120. The BAN server 120 processes signals from the sensors 132 to 138 to generate user's status information that is provided to the VAN 110 for controlling vehicular electronics devices. Specifically, the BAN server 120 analyzes signals from the sensors 132 to 138 to generate the user's status information. The user's status information may include the signals from the sensors 132 to 138 together with the analyzed results. The BAN server 120 then generates and transmits signals corresponding to the status information, preferably including the physiological signals and environmental signals, to the VAN 110 for controlling vehicular electronics devices located in or connected to the VAN 110.

The VAN 110 provides communication interface and control functions between the BAN server 120 and a plurality of vehicular electronics devices 240 to 252. The VAN 110 includes an interface module 210, a control module 220, a vehicle communication module 230, a memory 260, and a plurality of vehicular electronics devices 240 to 252 including a car computer 240, an audio device 242 (e.g., stereo system, CD player, MP3 player, etc), a window control device 244, an air conditioner system (A/C) 246, a seat control device 248, a navigation system 250, and a multimedia system 252 such as a digital mobile broadcasting (DMB) system.

The vehicular electronics devices 240 to 252 are exemplary only and may include other vehicular devices, vehicle systems, and vehicular control units such as the engine control unit, transmission control unit, drive train control unit, vehicle electrical control unit, door control unit, a car radio and/or a driver information system, a mobile radio communication unit, a diagnostic tool, a communication device for the exchange of the data (e.g., automatic toll collection or access authorization), a heating/air-conditioning system with corresponding control units, a GPS navigation device, or other vehicular devices, systems, or control units.

The interface module 210 in the VAN 110 provides an interface function by communicating with the BAN server 120 that transmits signals corresponding to the status information of a user. The interface function between the BAN server 120 and the interface module 210 may be implemented by employing short range wireless, e.g., Wi-Fi, communications such as through a Wireless Personal Area Network (PAN) using Bluetooth, Zigbee and other WPAN protocols, or using infrared or RF communications, without limitation thereto. However, wired communications between the VAN 110 and the BAN 120 may be used as well. Further, in one embodiment, the BAN server 120 may be connected to a docking station (not shown) which is connected to the interface module 210, so that the BAN server 120 can be inserted and removed as needed for connection to the VAN 110.

In selected embodiments, the interface module 210 may convert the status information signals from the BAN server 120 into digital data for use in the control module 220 and converting digital data from the control module 220 into signals for use in the BAN server 120. The control module 220 is coupled to memory 260 to receive the converted user information data from the interface module 210 and processes the data to generate device specific data signals based on the status information of the user. The control module 220 may retrieve a reference table from the memory 260, which associates the digital converted status information of the user with one or more vehicular electronics devices 240 to 252, thereby generating device specific data signals to control the vehicular electronics devices 240 to 252. The generated device specific signals are then provided to one or more associated vehicular electronics devices 240 to 252 to automatically control or adjust the setting of these devices. The control module 220 may send the device specific signal to the devices 240 to 252 by using a wired connection such as a serial interface.

For example, if the status information signals from the BAN server 120 indicates a higher than normal temperature and/or humidity, the control module 220 refers to the table indicating the association between the status information with the vehicular electronics devices to be controlled (the air conditioner system 246 and the window control system 244 in this example). In this example, the control module 220 may generate control data signals specific to the air conditioner system 246 to lower the temperature or to the window control system 244 to adjust (open or close) the window automatically. The control module 220 may send the control data signals through serial interface connection to each of the vehicular electronics devices.

The control module 220 may also provide the converted data to an external entity (e.g., service providers, hospitals, monitoring services, etc.) through the vehicle communication module 230 to allow the VAN 110 to provide an Internet connection to the BAN server 120. In one embodiment, the vehicle communication module 230 implements wireless broadband Internet such as WiBro, DMB (Digital Mobile Multimedia), or HSDPA (High Speed Downlink Pack Access) technology. In another embodiment, the communication module 230 provides wireless connectivity to mobile communications systems implementing wireless technologies such as GSM (Global System for Mobile Communications), GPS (Global Positioning System), 1x EV-DO (1x Evaluation Data Only), CDMA (Code Division Multiple Access), etc.
Additionally, the VAN 110 may provide data transmission between two or more BAN servers or mobile terminals.

As such, by using various kinds of wireless communication technologies, the user's physiological status information can be transmitted in real-time to a remote place. For example, the vehicle communication module 230 may also utilize such wireless communication capabilities to relay the physiological status data of a patient to a monitoring device located at a hospital while the patient is being transported to the hospital in the vehicle 270 equipped with the VAN 110 so that the patient's physical status can be continuously tracked in real time by a doctor in the hospital. As an other instance, the level of fatigue of a truck driver can be monitored by a sensor attached to the driver and the physiological status data can be transferred to a control center together with the condition of the truck via the vehicle communication module 230 installed in the truck such that the remote control center may monitor the condition of the driver and the truck simultaneously.

In addition to or instead of using the vehicle communication module 230, the VAN 110 may utilize the communication capabilities of the BAN server 120. In this embodiment, the BAN server 120 provides functions of a wireless communication interface to allow the VAN 110 to be connected to mobile communications systems such as GSM (Global System for Mobile Communications), GPS (Global Positioning System), 1x EV-DO (1x Evaluation Data Only), CDMA (Code Division Multiple Access), or other wireless standard or technology. In such cases, data from the VAN 110 is transferred to the BAN server 120 via the interface module 210 in the VAN 110. The BAN server 120 may also output the data on its display such as a liquid crystal display screen or a speaker (not shown).

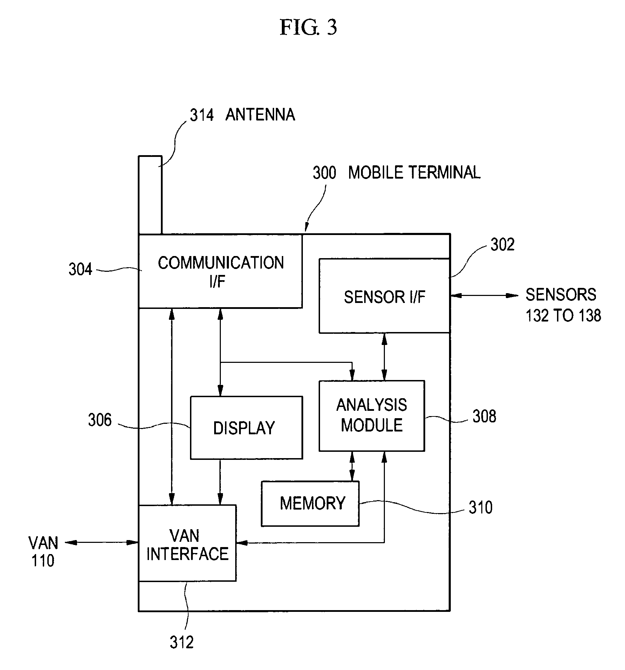

The BAN server 120 may be a terminal device installed on a vehicle or a mobile device carried by a user such as a mobile phone, PDA, or other mobile device. According to one embodiment, BAN server 120 is a mobile terminal 300 (FIG. 3).
The mobile terminal 300 receives physiological signals from the sensors 132 to 138 to monitor the physiological condition or status of a user and process the physiological signals to generate user's status information signals. The mobile terminal 300 provides the status information signals to the VAN 110 to allow the VAN 110 to control the vehicular electronics devices 240 to 252 based on the status information signals.
In the embodiment of FIG 3, the mobile terminal 300 includes a sensor interface 302, a communication interface 304, a display 306, an analysis module 308, a memory 310, a VAN interface 312, and an antenna 314.

The sensor interface 302 provides interface for the plurality of sensors 132 to 138, and is configured to receive physiological signals from the sensors 132 to 138 and extract physiological data and sensor ID's from the physiological signals to output physiological data to the analysis module 308. The sensor interface 302 may refer to a field value located at a certain position in the continuous physiological signals to verify the sensor ID and obtain the subsequent physiological data. The analysis module 308 analyzes the physiological data to determine user's status information indicating the user's physiological state or condition. For example, the analysis module 308 may determine the user's stress level based on one or more physiological signals such as EKG, beat-to-beat heart variability, heart rate, pulse rate, blood pressure, etc.

In addition to physiological data, the mobile terminal 300 may also receive and process environmental parameter signals from sensors that can detect air quality, sound level, light quality, ambient temperature near an individual, global positioning of the vehicle, etc. to generate environmental status information for the vehicle. The environmental status data may be used in combination with the physiological data to thereby allow the mobile terminal 300 to transmit a more precise status information data to the VAN 110. In one embodiment, the analysis module 308 may employ a table, which may be stored in memory 310 (e.g., flash memory) in the mobile terminal 300, to map physical and/or environmental parameters to the status information data to derive more precise status information. For example, the analysis module 308 may combine a physical parameter, e.g., a skin temperature of a driver and an environmental parameter, e.g., a room temperature to derive a precise body temperature of the driver. Specifically, the analysis module 308 may obtain a weighted sum of two parameter values to diagnose the body temperature.

Such table used to map physical and/or environmental parameters to the status information data is provided as Table 1 below. Table 1 includes exemplary types of user status information that are generated by the analysis module 308, types of physiological signals from sensors that can be used to generate the status information. Further, Table 1 may include device specific signals that are generated by the VAN 110 based on the status information, which may include the physiological or environmental signals, to be referred to control various vehicular electronics devices.

**TABLE 1**

| **Status information** | **Physiological Parameters** | **Device Specific Signals** |
|---|---|---|
| Body temperature | Skin temperature, core temperature, respiration rate | Vehicular window control signal to control the window 244, temperature control signal to control the A/C system 246, message display signal to control the LCD in car computer 240, audio control signal to control audio device 242 |
| Ovulation | Skin temperature, core temperature, oxygen consumption | Vehicular window control signal to control the window 244, audio control signal to control audio device 242 |
| Sleep onset/wake | Beat-to beat variability, heart rate, pulse rate, respiration rate, skin temperature, core temperature, heart flow, galvanic skin response, EMG, EEG, EOG, blood pressure, oxygen consumption | Vehicular window control signal to control window 244, audio control signal to control audio device 242, speed control signal to control the navigation system 250 |
| Calories burned | Heart rate, pulse rate, respiration rate, heat flow, activity, oxygen consumption | Audio control signal to control audio device 242, message display signal to control the LCD in the car computer 240 |
| Basal metabolic rate | Heart rate, pulse rate, respiration rate, heat flow, activity, oxygen consumption | Message display signal to the LCD in the car computer 240 |
| Basal temperature | Skin temperature, core temperature | Vehicular window control signal to control the window 244, temperature control signal to control the A/C system 246, message display signal to control the LCD in the car computer 240, audio control signal to control the audio device 242 |
| Activity level | Heart rate, pulse rate, respiration rate, heat flow, activity, oxygen consumption | Message display signal to control the LCD in the car computer 240, audio control signal to control audio device 242 |
| Stress level | EKG, beat-to-beat variability, heart rate, pulse rate, respiration rate, skin temperature, heat flow, galvanic skin response, EMG, EEG, blood pressure, activity, oxygen consumption | Message display signal to control the LCD in the car computer 240, audio control signal to control the audio device 242 |
| Relaxation level | EKG, beat-to-beat variability, heart rate, pulse rate, respiration rate, skin temperature, heat flow, galvanic skin response, EMG, EEG, blood pressure, activity, oxygen consumption | Message display signal to control the LCD in the car computer 240, audio control signal to control audio device 242, seat control signal to control the seat 248 |
| Maximum oxygen consumption rate | EKG, heart rate, pulse rate, respiration rate, heat flow, blood pressure, activity, oxygen consumption | Message display signal to control the LCD in the car computer 240, audio control signal to control audio device 242 |

With reference to Table 1, the analysis module 308 may collect some of the physiological data delivered from the sensor interface 302 to derive status information. The table may further specify the vehicular electronics devices in the vehicle 270 to be controlled corresponding to the status information derived from the physiological and environmental data through the analysis performed by the analysis module 308. For example, if some of the status information, e.g., sleep onset/wake, maximum oxygen consumption rate and the like in Table 1 indicate that the vehicle user is dozing off, the VAN 110 generates and transmits one or more device specific control signals, e.g., a vehicular window control signal, an audio control signal, a speed control signal to control corresponding vehicular electronics devices, e.g., the window 244, the audio device 242, the navigation system 250 so as to open the window, decelerate a vehicle speed, set a speed limitation, generate an audible warning, increase the speaker volume, display a warning on an LCD screen, etc.

In some embodiment, in order to determine a user's status (e.g., whether the driver is dozing off), suitable criteria or condition can be adopted in accordance with the unique physical characteristics of the driver. Specifically, a threshold value may be selected to be compared with each of the physiological parameters by reflecting the driver's personal physical characteristics. For example, referring to Table 1, the analysis module 308 determines whether the physiological parameters such as beat-to beat variability, heart rate, pulse rate, respiration rate, skin temperature, core temperature, heart flow, galvanic skin response, EMQ EEG, EOG, blood pressure, oxygen consumption, may exceed each of the predetermined thresholds so that if more than half of physiological parameters are more than the associated thresholds, the analysis module 308 may determine that the driver's status information associated with the above physiological data in Table 1 indicates that he is dozing off. The settings such as the thresholds can be input to the analysis module 308 by using an input device of the mobile terminal 300, e.g., a keypad (not shown),. In this manner, a user's status can be monitored to reflect the user's unique personal physiological characteristics or conditions.

Similar to the above example, if the analysis module 308 analyses that some of the physiological parameters such as skin temperature, core temperature, respiration rate of the driver as indicated by Table 1 are less than predetermined thresholds to sufficiently indicate that the user's status information (i.e., the user's body temperature in this example) has decreased to a specified level, then the analysis module 308 transmits the status information to the interface module 210 to allow the VAN 110 to determine a number of device specific signals associated with the status information (i.e., the user's body temperature in this example) as given by Table 1, and generates the associated device specific signals, such as a vehicular window control signal, a temperature control signal, message display signal, an audio control signal, thereby allowing the VAN 110 to control the window 244, the A/C system 246, the LCD in car computer 240, and the audio device 242. In this manner, the mobile terminal 300 may generate and transmit control signal to the VAN 110 to adjust the temperature setting in the air conditioning system 246 or to activate a seat heater accordingly so that the vehicle user can drive safely without the need to manually control the settings of the vehicular electronics devices in accordance with his or her physiological conditions.

With reference to Fig. 3, the analysis module 308 stores the user's status information, including the physiological and environmental data, to the memory 310 as they are generated or at periodic intervals. In one embodiment, the data is to be stored on an intermittent basis, and the analysis module 308 can be configured to determine statistical physiological data such as average, minimum, or maximum heart rate or respiration rate over a period of time (e.g., five minutes, ten minutes, etc.) to keep track of the user's physiological condition or state over time. The analysis module 308 provides such information as part of the user's status information to the VAN 110 via the VAN interface 312.

In communicating with the VAN 110, the mobile terminal 300 is configured to receive signals such as data, audio, or video signals from the VAN 110 for display on the display 306. The mobile terminal 300 may also function as a gateway to external networks by transmitting signals from the VAN 110 to the outside through a wireless network and/or the Internet.

The mobile terminal 300 may also transmit user's status information to a remote entity such as medical center together with the detected data via the network communication interface 304 and an antenna 314, thereby allowing the physician at the medical center to remotely check on the status of the individual's health condition. In selected embodiments, the mobile terminal 300 may transmit the status information repeatedly, e.g., on an hourly basis, to a third party that can give you advice upon detecting potential abnormal conditions of the user so that the analysis module 308 may use the advice to generate the status information. The third party may manage a remote server having a large amount of memory areas enough to store frequently sent status information so that the user or a third party may examine and analyze the health conditions over an extended period of time upon request or payment by the user. In this configuration, the user may subscribe a service for the health provider to monitor his or her health conditions based on the stored physiological signal. Further, the display 306 of the mobile terminal 300 may display the individual's status information from the analysis module 308 including the detected physiological and environmental data.

The analysis module 308 may be implemented using a microprocessor that may be adapted to execute suitable software program or instructions to perform the above mentioned algorithms. Such software program may be downloaded to the memory 310 remotely or locally via a read only memory ("ROM") or a flash memory connected to the mobile terminal 300. The software implementation of the analysis module 308 allows personalization of physiological parameter settings when analyzing the received physiological signals from the various sensors. In one embodiment of the software implementation of the analysis module 308, the user may specifically select a number of physiological/environmental parameters in Table 1 for each status information to be analyzed, and may also specify a threshold to compare with each value of physiological/environmental parameters in determining the associated status information. In this manner, an individual who has a history of heart disease in his or her family may be monitored more strictly with more sensitive physiological parameter settings.

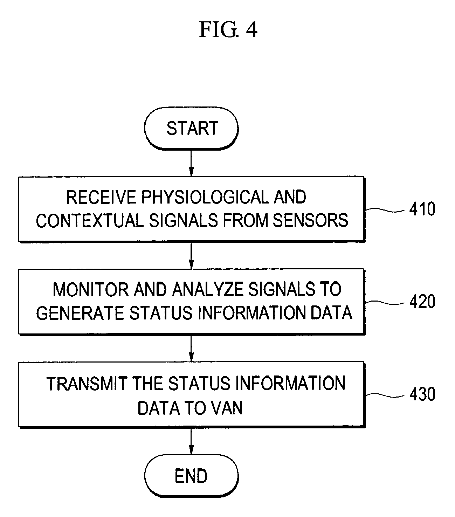

FIG 4 illustrates a method for controlling a plurality of vehicular electronics devices 240 to 252 by using the mobile terminal 300 in accordance with one embodiment of the present disclosure. At 410, the mobile terminal 300, preferably carried or worn by the user, receives physiological and/or environmental signals from the sensors 132 to 138. At 420, the analysis module 308 monitors and analyzes the received signals to generate status information data indicating the physiological condition or state of the user and environmental status of the vehicle, based on some of the physiological/environmental parameters in Table 1. For example, if the heart beat-to-beat variability is less than a predetermined threshold and the oxygen consumption is higher than a predetermined threshold, the analysis module 308 generates status information, i.e., sleep onset/wake in Table 1, indicating that the user is feeling drowsy. In addition to the physiological signals, a contextual signal, e.g., the air quality can be cooperatively used to derive such a condition. In one embodiment, the analysis module 308 monitors the status of the user and the vehicle even before the user enters the vehicle. In the alternative, the monitoring operation can be initiated after the user enters the vehicle; for example when he or she turns on the vehicle ignition when the analysis module 308 is set to dedicatedly operate and control the vehicular electronics devices.

At 430, the status information data is converted into analog signals and transmitted to the VAN 110 to control the vehicular electronics devices 240 to 252. Although the vehicular electronics devices 240 to 252 in the VAN 110 are controlled primarily according to the physiological status of the vehicle user with reference to the information given by Table 1, they may also be controlled by the VAN 110 based on a mobile terminal identification information such as, for example, a phone number, a user name, or a mobile identification number, either independently or in conjunction with the physiological status of the vehicle user. In this manner, the control settings for the vehicular electronics devices can be changed to suit a vehicle user carrying the identified mobile terminal based on predetermined information as well as the current physiological status of the vehicle user. Such an implementation may be advantageous especially when several people share the operating responsibilities of a vehicle, so that each particular user is provided with customized settings such as angle of mirrors, position of seats, etc. in accordance with the user's preference.

In one embodiment, the VAN 110 is configured to receive status information data from a plurality of BAN servers 120 such as mobile terminals 300, PDAs, etc. In order to operate with more than one BAN server, each of the BAN servers append an identification field for a predetermined ID stored in the memory of the mobile terminal to the status information data obtained from the servers associated with the specific BAN server so that the VAN 110 is configured to recognize a specific BAN server 120 by referring to the appended identification field. The VAN 110 may prioritize the multiple BAN servers by recognizing its identification field appended to the status information transmitted by the BAN servers to thereby customize each of the vehicular electronics devices installed in the vehicle according to the preference of the user associated with the BAN server 120 (e.g., carrying the BAN server 120 or mobile terminal 300). The BAN server 120 may actively transmit control signals in advance to the VAN 110 for such preference customization so that the VAN 110 may configure a certain priority table between the multiple BAN servers. In a typical example, the VAN 110 may select the BAN server associated with the driver as a top priority.

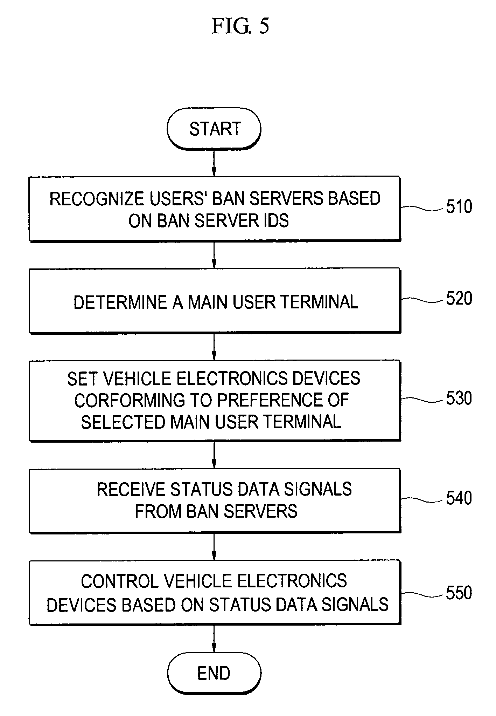

FIG 5 shows a flowchart of a method performed by the VAN 110 to control vehicular electronics devices 240 to 252 in accordance with one embodiment of the present disclosure. Initially, one or more vehicle users, each with a BAN server 120, enter the vehicle 270. Each BAN server 120 has a unique ID, which is pre-stored in the memory 310 of the BAN server and the memory 260 of the VAN 110, to identify the device. The BAN server IDs may be any data capable of uniquely identifying the BAN servers 120 such as a phone number or a mobile identification number in the case of mobile terminals. In order to recognize the BAN servers when the vehicle ignition is turned on, the VAN 110 is activated and proceeds to detect the BAN servers 120 located in the vehicle at 510 based on the BAN server IDs. For example, to detect the BAN servers 120, the interface module 210 of the VAN 110 broadcasts a query signal to the BAN servers 120 in the vehicle. Each of the BAN servers 120 located and active in the vehicle then responds to the query signal by transmitting a response signal to the interface module 210 along with its BAN server ID (which may be embedded in the response) to indicate its active status.

In one embodiment, the VAN 110 stores the BAN server IDs with an assigned priority level to identify a main user (e.g., driver) and users of other priority levels. In addition, the VAN 110 may store customized settings for the vehicular electronics devices 240 to 252 for each of the BAN servers according to the associated IDs. To this end, the VAN 110 may configure and store a lookup table specifying a relation between the BAN server IDs and customized settings for the vehicular electronics devices 240 to 252. When the VAN 110 detects only one BAN server, i.e., when it receives a response signal and BAN server ID from only one BAN server 120, it automatically configures the settings of the vehicular electronics devices according to the pre-stored preference settings of the associated BAN server 120, e.g., by searching the pre-configured lookup table or by using a default values.

When two or more BAN servers 120 respond to the query signal, the control module 220 of the VAN 110 determines, in operation 520, a main BAN server according to the assigned priority level among the users based on the BAN server IDs. The extracted mobile terminal IDs are compared to the set of IDs stored previously in the memory 260 of the VAN 110 to identify and verify the BAN servers.
The main user may be determined, in one embodiment, according to the previously stored priority levels assigned to each BAN server 120 associated with a user. For example, when members of a family are registered as possible drivers of the vehicle, the BAN server ID of the mother may be stored in VAN 110 with the highest rank in the matching table. In another embodiment, the BAN server 120 of a user that is inserted into a docking station of the VAN 110 is automatically assigned to be the main user by default. In yet another embodiment, the main user may be set manually by pressing a predetermined button (not shown) on the BAN server 120 associated with the main user. Once the main user is determined, the settings of the vehicular electronics devices 240 to 252 are customized at 530 according to customized settings of the main user by accessing the stored data in the VAN 110's memory 260, e.g., in the form of the lookup table. In the case above where the mother is determined to be the main user, the vehicle is customized to her preference settings. Alternatively, the vehicular electronics devices may instead be customized to the preference of the person sitting in the driver's seat. If no preference settings are found in the memory 260, default values either may be used to set the vehicular electronics devices or no customization is performed until manually customized by a user.

At 540, the VAN 110 receives status data signals indicating users' conditions from each of the BAN servers 120, each of which receives physiological signals of a user through one or more associated sensors. Preferably, the physiological data signals transmitted by the sensors include an identifier to identify the physiological data signals. In this arrangement, the physiological status of each of the users is analyzed and monitored by his or her BAN server and communicated to the VAN 110.

At 550, the control module 220 of the VAN 110 processes the received status data to generate device specific control data to control the vehicular electronics devices, e.g., with reference to the association between the status information and the device specific data given by Table 1. In one embodiment, the driver's physiological status is assigned the highest priority level for controlling the vehicular electronics devices.
For example, if the driver's BAN server 120 transmits status data signals to the VAN 110 that indicates that the driver is shivering, then the control module 220 controls the vehicular electronics devices by closing windows or activating the heater in the vehicle.

Aspects of the subject matter described herein are set out in the following numbered clauses:
1. A vehicle network system for controlling at least one vehicular electronics device, comprising:
   at least one sensor adapted to generate physiological signals of a user if connected to the user;
   a network server configured to receive and process the physiological signals from the at least one sensor to generate status data indicating a physical condition of the user; and
   a network device configured to receive the status data from the network server and to control the at least one vehicular electronics device based on the status data.
2. The vehicle network system of Clause 1, wherein the network server is a mobile terminal.
3. The vehicle network system of Clause 2, wherein the network server is a mobile phone.
4. The vehicle network system of Clause 1, wherein each of the at least one sensor transmits a unique sensor ID to the network server for identifying the physiological signals.
5. The vehicle network system of Clause 1, wherein the network server transmits a unique server ID to the vehicle network device for identifying the network server.
6. The vehicle network system of Clause 5, wherein the server ID includes a phone number and a mobile identification number.
7. The vehicle network system of Clause 1, wherein the network device assigns a priority level to the network server.
8. The vehicle network system of Clause 1, wherein the network device stores precustomized settings of the at least one vehicular electronics device for the network server.
9. The vehicle network system of Clause 8, wherein the network device controls the at least one vehicular electronics device based on the precustomized settings.
10. The vehicle network system of Clause 5, wherein the network device controls the at least one vehicular electronic device based on the unique server ID.
11. The vehicle network system of Clause 1, wherein the network device generates device specific signals for the at least one vehicular electronics devices.
12. The vehicle network system of Clause 1, wherein the network server provides the physiological signals to a third party entity.
13. The vehicle network system of Clause 1, wherein the network server provides the physiological signals to a health provider.
14. The vehicle network system of Clause 1, wherein the network server provides the physiological signals to a service provider that provides health information to the user based on subscription to a service provided by the service provider.
15. The vehicle network system of Clause 1, wherein the network server communicates with the network device via a wireless connection.
16. The vehicle network system of Clause 15, wherein the wireless connection includes Bluetooth, Zigbee, infrared and RF communication.
17. The vehicle network system of Clause 1, wherein network server communicates with the network device via a wired connection.
18. The vehicle network system of Clause 17, wherein the wired connection includes a docking station disposed in the network device, the network server capable of being inserted into the docking station.
19. A vehicle network system for controlling vehicular electronics devices, comprising:
   a plurality of network servers each configured to receive and process physiological signals from one or more sensors to generate status data indicating the physical condition of a user; and,
   a vehicle network device configured to control one or more vehicular electronics devices based on the status data from the plurality of network servers.
20. The vehicle network system of Clause 19, wherein each of the network servers is a mobile terminal.
21. The vehicle network system of Clause 20, wherein each of the network servers is a mobile phone.
22. The vehicle network system of Clause 19, wherein each of the one or more sensors transmits a unique sensor ID to the associated network server for identifying the associated physiological signals.
23. The vehicle network system of Clause 19, wherein each of the network servers transmits a unique server ID to the vehicle network device for identifying the network server.
24. The vehicle network system of Clause 23, wherein the vehicle network device controls the one or more vehicular electronics devices based on the unique server IDs.
25. The vehicle network system of Clause 23, wherein the vehicle network device assigns a priority level to each of the unique server IDs and wherein the vehicle network device controls the one or more vehicular electronics devices based on the priority levels of the unique server IDs.
26. The vehicle network system of Clause 19, wherein the vehicle network device generates device specific signals for controlling the one or more vehicular electronics devices.
27. The vehicle network system of Clause 23, wherein the server ID includes a phone number and a mobile identification number.
28. The vehicle network system of Clause 19, wherein the network servers provide the physiological signals to a third party entity.
29. The vehicle network system of Clause 19, wherein the network servers provide the physiological signals to a health provider.
30. The vehicle network system of Clause 19, wherein the network servers provide the physiological signals to a service provider that provides health information to the users based on subscription to a service provided by the service provider.
31. The vehicle network system of Clause 25, wherein the vehicle network device determines a main network server based on the priority level of each of the unique server IDs.
32. The vehicle network system of Clause 31, wherein the vehicle network device determines one of the network servers of which the server ID indicates a driver as the main network server.
33. The vehicle network system of Clause 19, wherein the vehicle network device determines a network server transmitting a control signal indicating a driver to the vehicle network device as a main network server.
34. The vehicle network system of Clause 19, wherein the vehicle network device stores precustomized settings of the one or more vehicular electronics devices for the network servers.
35. The vehicle network system of Clause 34, wherein the vehicle network device controls the at least one or more vehicular electronics device based on the precustomized settings.
36. The vehicle network system of Clause 33, wherein the vehicle network device customizes settings of the at least one or more vehicular electronics device based on the control signal.
37. A method for controlling vehicular electronics devices, comprising:
   processing sensed physiological signals of a user to generate status data indicating a physical condition of the user;
   generating control signals specific to one or more vehicular electronics devices based on the status data; and
   providing the device specific control signals to the one or more vehicular electronics devices to control the one or more vehicular electronics devices.
38. The method of Clause 37, wherein the physiological signals are generated by one or more sensors.
39. The method of Clause 38, wherein each of the sensors transmits a unique sensor ID for identifying the associated physiological signals.
40. The method of Clause 37, wherein the physiological signals are processed by a mobile terminal to generate the status data indicating the physical condition of the user and wherein the mobile terminal transmits a unique terminal ID for identifying the mobile terminal to the one or more vehicular electronics devices.
41. The method of Clause 40, wherein the one or more vehicular electronics devices are controlled based on the unique terminal ID.
42. The method of Clause 40, wherein the terminal ID includes a phone number and a mobile identification number.
43. The method of Clause 40, further comprising assigning a priority level to the unique terminal ID.
44. A method for monitoring a health status of a user, comprising:
   processing sensed physiological signals of a user to generate status data indicating a physical condition of the user;
   transmitting the physiological signals to a service provider;
   receiving status information on the health conditions of the user from the service provider; and
   generating control signals specific to one or more vehicular electronics devices based on the status information.
45. The method of Clause 44, further comprising
   providing the device specific control signals to one or more vehicular electronics devices to control the one or more vehicular electronics devices.
46. The method of Clause 44, wherein the physiological signals are generated by one or more sensors
47. The method of Clause 46, wherein each of the sensors transmits a unique sensor ID for identifying the associated physiological signals.
48. The method of Clause 44, wherein the physiological signals are processed by a mobile terminal.
49. The method of Clause 48, wherein the mobile terminal transmits the physiological signals to the service provider, thereby allowing the user to monitor the health condition based on a subscription to a service provided by the service provider.
50. An apparatus for controlling at least one vehicular electronics device by using at least one sensor, comprising:
   a sensor interface configured to receive physiological signal from at least one sensor associated with a user;
   a processing module configured to process the physiological signals to generate status data indicating a physical condition of the user; and
   a vehicle interface configured to communicate with a vehicle network device based on the status data.
51. The apparatus of Clause 50, wherein the apparatus is a mobile terminal.
52. The apparatus of Clause 51, wherein the apparatus is a mobile phone.
53. The apparatus of Clause 50, wherein the vehicle interface transmits the status data to the vehicle network device to control at least one vehicular electronics device connected to the vehicle network device.
54. The apparatus of Clause 50, wherein the interface module communicates with the vehicle network device by a wireless connection.
55. The apparatus of Clause 54, wherein the wireless connection includes Bluetooth, Zigbee, infrared and RF communication.
56. The apparatus of Clause 54, wherein the interface module communicates with the vehicle network device by a wired connection.
57. The apparatus of Clause 56, wherein the wired connection includes a docking station of the vehicle, the device capable of being inserted into the docking station.
58. The apparatus of Clause 50, further comprising:
   a memory to store the status data.
59. The apparatus of Clause 50, further comprising:
   a communication module configured to communicate to a wireless network.
60. The apparatus of Clause 59, wherein the communication module transmits the status data and/or the physiological signals to a third party entity via the wireless network.
61. The apparatus of Clause 59, wherein the communication module transmits the status data and/or the physiological signals to a service provider that provides health information to the user based on subscription to a service provided by the service provider.
62. The apparatus of Clause 50, further comprising:
   a display configured to display the status data.
63. The apparatus of Clause 50, wherein the sensor interface receives a unique sensor ID for identifying the physiological signals for each of the at least one sensor.
64. The apparatus of Clause 50, wherein the vehicle interface transmits a unique apparatus ID to the vehicle network device for identifying the apparatus.
65. A vehicle, comprising:
   a vehicular network system including,
   an interface configured to receive status information of a user from an external device;
   at least one vehicular electronics device disposed in the vehicle; and
   a controller configured to control the at least one vehicular electronics device based on the status information.
66. The vehicle of Clause 65, wherein the interface receives a unique device ID for identifying the external device from the external device.
67. The vehicle of Clause 66, wherein the controller controls the at least one vehicular electronics device based on the unique device ID.
68. The vehicle of Clause 65, wherein the controller generates device specific signals for the at least one vehicular electronics device based on the status information.
69. The vehicle of Clause 65, wherein the interface communicates with the external device by a wireless connection.
70. The vehicle of Clause 69, wherein the wireless connection includes Bluetooth, Zigbee, infrared and RF communication.
71. The vehicle of Clause 65, wherein the interface module communicates with the external device by a wired connection.
72. The vehicle of Clause 71, wherein the wired connection includes a docking station of the vehicle, the external device capable of being inserted into the docking station.
73. The vehicle of Clause 65, further comprising at least one sensor adapted to generate environmental signals indicating a status of the vehicle.
74. The vehicle of Clause 73, wherein the controller controls the at least one vehicular electronics device based on the environmental signals.

While the present invention has been shown and described with respect to specific embodiments, those skilled in the art will recognize that various changes and modifications may be made without departing from the spirit and scope of the invention as defined in the appended claims.

## Claims

1. A vehicle network system for controlling at least one vehicular electronics device, comprising:
at least one sensor adapted to generate physiological signals of a user if connected to the user;
a network server configured to receive and process the physiological signals from the at least one sensor to generate status data indicating a physical condition of the user; and
a network device configured to receive the status data from the network server and to control the at least one vehicular electronics device based on the status data.

2. The vehicle network system of Claim 1, wherein the network server is a mobile terminal.

3. The vehicle network system of Claim 2, wherein the network server is a mobile phone.

4. The vehicle network system of any one of the preceding Claims, wherein each of the at least one sensor transmits a unique sensor ID to the network server for identifying the physiological signals.

5. The vehicle network system of any one of the preceding Claims, wherein the network server transmits a unique server ID to the vehicle network device for identifying the network server.

6. The vehicle network system of Claim 5, wherein the server ID includes a phone number and a mobile identification number.

7. The vehicle network system of any one f the preceding Claims, wherein the network device assigns a priority level to the network server.

8. The vehicle network system of any one of the preceding Claims, wherein the network device stores precustomized settings of the at least one vehicular electronics device for the network server.

9. The vehicle network system of Claim 8, wherein the network device controls the at least one vehicular electronics device based on the precustomized settings.

10. The vehicle network system of Claim 5, wherein the network device controls the at least one vehicular electronic device based on the unique server ID.

11. The vehicle network system of any one of the preceding Claims, wherein the network device generates device specific signals for the at least one vehicular electronics devices.

12. The vehicle network system of any one of the preceding Claims, wherein the network server provides the physiological signals to a third party entity.

13. The vehicle network system of any one of the preceding Claims, wherein the network server communicates with the network device via at least one of a wireless connection or a wired connection.

14. The vehicle network system of Claim 13, wherein a said wired connection includes a docking station disposed in the network device, the network server capable of being inserted into the docking station.

15. The vehicle network system of any one of the preceding Claims, comprising:
a plurality of said network servers, each configured to receive and process physiological signals from one or more sensors to generate status data indicating the physical condition of a user; wherein
the vehicle network device is configured to control the one or more vehicular electronics devices based on the status data from the plurality of network servers.

16. The vehicle network system of Claim 15, wherein each of the network servers transmits a unique server ID to the vehicle network device for identifying the network server.

17. The vehicle network system of Claim 16, wherein the vehicle network device assigns a priority level to each of the unique server IDs and wherein the vehicle network device controls the one or more vehicular electronics devices based on the priority levels of the unique server IDs.

18. The vehicle network system of Claim 17, wherein the vehicle network device determines a main network server based on the priority level of each of the unique server IDs.

19. The vehicle network system of Claim 18, wherein the vehicle network device determines one of the network servers of which the server ID indicates a driver as the main network server.

20. The vehicle network system of any one of Claims 16 to 19, wherein the vehicle network device determines a network server transmitting a control signal indicating a driver to the vehicle network device as a main network server.

21. A vehicle, comprising the vehicle network system of any one of Claims 1 to 20.

22. A method for controlling vehicular electronics devices using the vehicle network system according to any one of Claims 1 to 20, comprising:
processing sensed physiological signals of a user to generate status data indicating a physical condition of the user;
generating control signals specific to one or more vehicular electronics devices based on the status data; and
providing the device specific control signals to the one or more vehicular electronics devices to control the one or more vehicular electronics devices.

23. A method for monitoring a health status of a user using the vehicle network system of any one of Claims 1 to 20, comprising:
processing sensed physiological signals of a user to generate status data indicating a physical condition of the user;
transmitting the physiological signals to a service provider;
receiving status information on the health conditions of the user from the service provider; and
generating control signals specific to one or more vehicular electronics devices based on the status information.
